Europäisches Patentamt

European Patent Office

Office européen des brevets

(19)

(11) **EP 0 491 739 B1**

(12) **EUROPÄISCHE PATENTSCHRIFT**

(45) Veröffentlichungstag und Bekanntmachung des
Hinweises auf die Patenterteilung:
**17.12.1997 Patentblatt 1997/51**

(21) Anmeldenummer: **90912867.0**

(22) Anmeldetag: **06.09.1990**

(51) Int Cl.[6]: **C12N 9/10**, C12N 9/12,
C12N 9/14, C12P 19/28,
C07F 9/6561

(86) Internationale Anmeldenummer:
**PCT/DE90/00678**

(87) Internationale Veröffentlichungsnummer:
**WO 91/04322 (04.04.1991 Gazette 1991/08)**

(54) **Herstellung von Desoxyribonucleotiden und Phosphatestern von Nucleosiden mit Hilfe multifunktioneller Enzyme**

MULTI-FUNCTIONAL ENZYMES INCLUDING DERIVABLE 2',3'-DIDESOXYRIBOFURANOSIDE TRIPHOSHPATES

ENZYMES MULTIFONCTIONNELLES ET TRIPHOSPHATES DERIVABLES DE 2',3'-DIDESOXYRIBOFURANOSIDE

(84) Benannte Vertragsstaaten:
**AT BE CH DE DK ES FR GB IT LI LU NL SE**

(30) Priorität: **12.09.1989 DE 3930441**
**28.06.1990 DE 4020529**

(43) Veröffentlichungstag der Anmeldung:
**01.07.1992 Patentblatt 1992/27**

(60) Teilanmeldung: **97115112.1**

(73) Patentinhaber: **Havlina, Roxana-Maria**
**14513 Teltow (DE)**

(72) Erfinder: **Havlina, Roxana-Maria**
**14513 Teltow (DE)**

(74) Vertreter: **Vossius, Volker, Dr. et al**
**Dr. Volker Vossius,**
**Patentanwaltskanzlei - Rechtsanwaltskanzlei,**
**Holbeinstrasse 5**
**81679 München (DE)**

(56) Entgegenhaltungen:
- **Biological Abstracts, Band 58, Nr. 4, 1974, (Philadelphia, PA, US), S. JYSSUM et al.: "Search for Thymidine Phosphorylase Nucleoside Deoxyribosyltransferase and Thymidine Kinase in Moraxella Acinetobacter, and Allied Bacteria" siehe seite 1882, Zusammenfassung Nr. 17637 & Acta Pathol. Microbiol. Scand. Sect. B Microbiol. Immunol., 82(1), 57-66, 1974**
- **Biochemical and Biophysical Research Communications, vol. 155, No. 2 15**
- **September 1988, Academic Press, Inc., (Duluth, US), D.A. CARSON et al.: "Synthesis of 2',3'-Dideoxynucleosides by Enzymatic Transglycosylation", seiten 829-834 siehe das ganzen artikel**
- **CHEMICAL ABSTRACTS, Band 83, Nr. 7, 18. August 1975, (Columbus, Ohio, US) J. HOLGUIN et al.: "Trans-N-Deoxyribosylase. Purification by Affinity Chromatography and and Characterization" siehe seite 199, Zusammenfassung Nr. 55084z & Eur. J. Biochem., 1975, 54(2), 505-14**
- **CHEMICAL ABSTRACTS, Band 98, Nr. 21, 23. Mai 1983, (Columbus, Ohio, US), M.C. HUANG et al.: "Formation of 3-(2'-Deoxyribofuranosyl) and 9-(2'-Deoxyribofuranosyl) Nucleosides of 8-Substituted Purines by Nucleoside Deoxyribosyltransferase" siehe seite 326, Zusammenfassung Nr. 175460a & Arch. Biochem. Biophys. 1983, 222(1), 133-44**

EP 0 491 739 B1

## Beschreibung

Es ist bekannt, daß der Mikroorganismus *Lactobacillus leichmannii* und *Lactobacillus helveticus* zwei Nucleosid-Desoxyribosyltransferasen, DRT I (noch ohne EC-Nummer) und DRT II (EC 2.4.2.6), besitzt; Min-Chi Huang et al., *Archives of Biochemistry and Biophysics,* Bd. 222 (1983), 133-144 und Holguin et al., Eur. J. Biochem. 1975, 54(2), 505-514. DRT I und DRT II katalysieren den Transfer der 2'-Desoxyribose von einem Pyrimidin- oder Purinnucleosid auf eine andere Pyrimidin- oder Purinbase nach folgenden Reaktionsschemata:

$$\text{dRib-Pur + Pur} \rightleftarrows \text{dRib-Pur' + Pur} \qquad\qquad \text{DRT I}$$

$$\text{dRib-Pyr(Pur) + Pyr'(Pur')} \rightleftarrows \text{dRib-Pyr'(Pur') + Pyr(Pur)} \qquad\qquad \text{DRT II}$$

$$\text{dRib-Pyr + Pur} \rightleftarrows \text{dRib-Pur + Pyr} \qquad\qquad \text{DRT II}$$

Abkürzungen:

Pur :    Purin-Base
Pyr :    Pyrimidin-Base
dRib:    2'-Desoxyribose

Überraschenderweise wurde während des Aufreinigungsvorgangs der bereits bekannten Nucleosid-Desoxyribosyltransferasen, hier V 1 und V 2 genannt, aus *Lactobacillus leichmannii* eine dritte Nucleosid-Desoxyribosyltransferase V 3 gefunden, die ebenfalls folgende Reaktion katalysiert:

$$\text{dRib-Pur + Pur} \rightleftarrows \text{dRib-Pur' + Pur}$$

Die Aufreinigung der drei Enzyme erfolgte erfindungsgemäß über zwei Vorreinigungsschritte und eine Ionenaustauschchromatographie, wobei die drei Proteine mit guter Auflösung voneinander getrennt wurden. Jedes Protein wurde weiter über eine Affinitätschromatographie aufgereinigt.

Auf diese Weise wurden die Enzyme V 1, V 2, und V 3 bis zur Homogenität aufgereinigt.

V 1, V 2 und V 3 sind allosterische Enzyme, welche Aggregate bilden. Bei steigender Ionenstärke oder Lösungsmittelkonzentration oder beim Lyophilisieren können sie sich zu größeren Aggregaten (Dimeren, Trimeren usw.) zusammenlagern, die elektrophoretisch nachgewiesen worden sind.

Sowohl dieses neue Enzym V3 als auch die bekannten besitzen überraschenderweise nicht nur Desoxyribosyltransferase-Aktivität, sondern auch Nucleotid-Kinase-, Ribonucleotid-Reduktase-, Adenosin-Desaminase- und DNA-Polymerase-Aktivität.

Alle diese Aktivitäten liegen auf der gleichen Polypeptidkette und sind durch die bekannten präparativen und analytischen biochemischen Methoden wie Affinitätschromatographie, Elektrophorese, Isofokusierung (isoelektrische Fokusierung) nicht trennbar. Alle diese Aktivitäten haben gemeinsame Inhibitoren und Aktivatoren, was beweist, daß die aktiven Zentren für alle diese Funktionen Bestandteile eines Polyproteins sind.

Transferase-Aktivität

V 3 ist in der Lage, den Transfer eines 2'-Desoxy- oder 2',3'-Didesoxyribofuranosylrestes von einem 2'-Desoxy- oder 2',3'-Didesoxyribonucleosid auf eine Purinbase zu katalysieren, wobei 2'-Desoxy- oder 2',3'-Didesoxyribofuranoside gebildet werden (Beispiel 2 und 3).

Die enzymatische Aktivität wurde durch folgende zwei Methoden bestimmt:

**Direktes spektrophotometrisches Verfahren:**

Dieses Verfahren beruht auf der größeren Extinktion des synthetisierten 6-Chlor-2'-desoxyguanosins bei 305 nm im Vergleich zur eingesetzten Akzeptorbase 6-Chlorguanin ($\varepsilon_{305\ nm} = 760\ M^{-1} \times cm^{-1}$; $\varepsilon$ = Molarer Extinktionskoeffizient). Im Reaktionsansatz werden 1 mM 2'-Desoxyadenosin oder 2'-Desoxyinosin und 0,2 mM 6-Chlorguanin verwendet.

Eine Aktivitätseinheit wurde definiert als Absorptionssteigerung von 0,01 Absorptionseinheiten bei 305 nm/ 37 °C/min/ml.

**Methode nach R. Cardinaud (*Methods in Enzymology*, Bd. LI):**

In einem Reaktionsansatz mit 0,3 M Phosphatpuffer pH 6,0, 1 mM 2'-Desoxyinosin, 1 mM Akzeptorbase, 50 µl Xanthin-Oxidase [10 mg/ml in 3,2 M $(NH_4)_2SO_4$ von Boehringer Mannheim, verdünnt 1:10 mit 2M $(NH_4)_2SO_4$], wird das Hypoxanthin, welches nach Übertragung der Desoxyribose aus 2'-Desoxyinosin entsteht, vom Hilfsenzym zu Xanthin und weiter zu Harnsäure oxidiert. Harnsäure besitzt ein Absorptionsmaximum bei 290 nm.

Die erste Methode besitzt den Vorteil, daß man das Nucleosid beliebig variieren kann, während bei der zweiten Methode verschiedene Akzeptorbasen getestet werden können.

Analytisch wurde der Reaktionsverlauf der Transferreaktion, aber auch der Phosphorylierung, Reduktion, Desaminierung und Polymerisation mittels HPLC-Chromatographie verfolgt. Die Reversed-Phase-Matrix bestand aus Octadecylsilan (Nucleosid 300-5 C $_{18}$), wobei als Elutionsmittel $KH_2PO_4$ 50 mM verwendet wurde und die Methanolkonzentration, je nach Hydrophobizität des Produktes, zwischen 2,5 % bis 50 % variierte.

V 3 erkennt insbesondere 1-, 7-, 8-, bzw. 3-substituierte Purine als Akzeptorbasen, während V2 vorwiegend die 2- bzw. 6-substituierten Purine erkennt.

Die 2', 3' -Didesoxyribofuranoside können durch die Nucleotid-Kinase-Aktivität bis zu den entsprechenden Triphosphaten phosphoryliert werden (Beispiel 5).

Nucleotid-Kinase-Aktivität

Die Enzyme V 1, V 2 und V 3 aus *Lactobacillus leichmannii* katalysieren die Übertragung einer anorganischen Phosphatgruppe von einem Nucleotid (Phosphatdonor) auf ein Nucleosid oder Nucleotid (Akzeptor).

Die Phosphatakzeptoren können stufenweise über die Monophosphat- und Diphosphat- zu den Triphosphat-Desoxynucleotiden bzw. Didesoxynucleotiden bzw. Nucleotiden bzw. deren Analoga phosphoryliert werden (Beispiel 4 und 5).

Das aktive Zentrum der Kinasen ist hitzeempfindlich. Eventuelle Hitzedenaturierungsschritte bei der Aufreinigung der Enzyme sind zu vermeiden, und die Synthesen sind bei 35°C durchzuführen.

Die Substratspezifizität von V 1, V 2 und V 3 betreffend die Akzeptoren der Phosphorylierungs-Reaktion sind folgende:

| Enzym | Substrat |
|---|---|
| V 1 | dC, dG, dA, die entsprechende Nucleotide und Analoga |
| V 2 | dG, dA, dC, dT, entsprechende Nucleotide und Analoga |
| V 3 | dT, dC, dG, dA, entsprechende Nucleotide und Analoga |

Die Effizienz der verschiedenen Phosphatdonoren (Nucleotide) in Abhängigkeit vom Akzeptor ist unterschiedlich und kann aus der folgenden Tabelle entnommen werden:

| Substrat | Donor | Effizienz (%) |
|---|---|---|
| dT | dATP | 100 |
| dG | ATP | 52 |
| dC | CTP | 18 |
| dA | dCTP | 100 |
| | dGTP | 76 |
| | CTP | 45 |
| | ATP | 39 |
| | GTP | 20 |

Durch das breite Substratspektrum von V 1, V 2 und V 3 ist eine große Vielfalt an Akzeptoren möglich.

Ribonucleotid-Reduktase-Aktivität

Die Enzyme V 1, V 2 und V 3 können die Reduktion von Nucleotiden in Gegenwart eines Coenzyms und eines Effektors katalysieren.

Als Substrat für die Reduktion können ATP, CTP, GTP und UTP sowie deren Di- und/oder Monophosphate sowie deren Analoga eingesetzt werden (Beispiel 6).

Für effiziente Reaktionsgeschwindigkeiten ist sowohl die Anwesenheit der Effektoren als auch des Coenzyms B 12 unbedingt nötig.

Wenig Reduktionen finden in Abwesenheit der Effektoren statt. Das Coenzym B12 ist kommerziell erhältlich, z.B. bei der Firma Fluka AG, BRD. Coenzym B 12 Konzentrationen über 50 μM oder Konzentrationen von 15-20 μM in Anwesenheit von 10 μM 5'-Desoxyadenosin inhibieren alle Aktivitäten der multifunktionellen Enzyme.

Folgende Effektoren sind bei der Reduktionsreaktion nötig:

| Substrat | Effektor |
|----------|----------|
| ATP | dGTP |
| CTP | dATP |
| UTP | dCTP |
| GTP | dTTP |

Eine Reduktion auf der Mono- und Diphosphat-Stufe ist ebenfalls möglich.

Adenosin-Desaminase-Aktivität

Die Enzyme V 1, V 2 und V 3 katalysieren die Hydrolyse der Aminogruppe von Nucleotiden, Nucleosiden, Purin- und Pyrimidinbasen.

Um die Desaminierung erfolgreich durchzuführen, muß der Reaktionsansatz bei 37°C über längere Zeit inkubiert werden, bis die Reaktion einsetzt. Beispielsweise wird aus 2'-Desoxycytidin das 2'-Desoxyuridin und aus 2',3'-Didesoxyadenosin das 2',3'-Didesoxyinosin gebildet (Beispiel 7 und 8).

Diese sogenannte lag-Phase (in den ersten Stunden findet keine Reaktion statt) ist ein typisches Verhalten der multifunktionellen Enzyme und wird auf eine Konformationsänderung zurückgeführt.

Nucleosidase-Aktivität

Die Enzyme V 1, V 2 und V 3 besitzen auch die Fähigkeit, die glycosidische Bindung der Nucleoside zu hydrolysieren. Die Hydrolyse zeigt genau wie die Desaminierungsreaktion eine lag-Phase.

DNA-Polymerase-Aktivität

Die multifunktionellen Enzyme aus *Lactobacillus*, V 1, V 2 und V 3, die gleichzeitig Transferase-, Reduktase-, Desaminase- und Kinase-Aktivität besitzen, vermögen auch die Umsetzung von Desoxyribonucleotiden zu katalysieren, wobei Homo- oder Heteropolymere entstehen. Das ist eine de novo-Polymerisations-Aktivität. Die Verknüpfung der Nucleotide erfolgt auf allen Phosphorylierungsstufen. Sowohl Triphosphat- als auch Diphosphat- und Monophosphat-Desoxynucleoside werden in Polymere eingebaut. Die de novo-Polymerisation zeigt auch eine lag-Phase von 20 bis 70 Stunden (Beispiel 9).

J.P. Durham und D.H. Ives haben sich mit der Desoxynucleosid-Kinase-Aktivität der multifunktionellen Enzyme beschäftigt, konnten jedoch das Endprodukt nicht identifizieren (1).

Eine Multifunktionalität der Desoxyribonucleosid-Kinasen wurde schon 1977 (2,3,4) aus kinetischen Untersuchungen abgeleitet. Es wurde festgestellt, daß die Phosphorylierung von Desoxyadenosin und von Desoxyguanosin bei zwei verschiedenen aktiven Zentren eines einzigen Polypeptids stattfindet (5).

Diese Polyproteine können als eine Polypeptidkette (Produkt von einem einzigen Gen) mit zwei oder mehreren aktiven Zentren definiert werden (6).

Von den drei multifunktionellen Enzymen aus *Lactobacillus*, V 1, V 2, V 3, zeigen nur V 2 und V 3 de novo-Polymerase-Aktivität, aber alle drei katalysieren die Replikation eines Templates wie z.B. Poly [d(A-T)] (Beispiel 10 und 11).

Die drei Enzyme aus *Lactobacillus* katalysieren mit unterschiedlichen Effizienzen die Replikation verschiedener natürlicher und synthetischer DNAs: doppelsträngige, einzelsträngige, ringförmige, denaturierte. Mit einer Effizienz von 2-3 % der Einbaurate der Desoxyribonucleotide werden die Ribonucleotide polymerisiert.

Es ist bekannt, daß die DNA-Polymerasen sechs hochkonservierte Sequenzen besitzen (9,11,15,16). Deswegen wurden die entsprechenden Enzyme von anderen Organismen auf die gleichen Aktivitäten getestet.

Aufgereinigt bis zu Homogenität nach einer modifizierten Methode von Pfrogner (14) zeigte die Adenosin-Desaminase aus Kalbsmilz (Fa. Worthington Biochemical Corporation, Lot Nummer 59P412) ähnliche Eigenschaften. Das Enzym wurde 500 fach durch isoelektrische Fällung, Ammoniumsulfat-Fällung, Chromatographien an Bio-Rex-70, DEAE-Sephadex A-50, SE-Sephadex C-50 (wobei die letzten zwei Schritte wiederholt wurden) aufgereinigt.

Das multifunktionelle Enzym aus Kalbsmilz katalysiert die Reduktion von ADP und UDP (in Anwesenheit von Effektoren und NADPH), die Phosphorylierung von dAMP und dCMP (mit einem entsprechenden Phosphatdonor), die de novo-Polymerisation der Nucleotide und die Replikation eines Templates (Beispiel 12).

Die *E. coli* DNA-Polymerase I (Pharmacia, Bestellnummer 27-0626-02, *E. coli* CM Stamm 5197), ein bekanntes multifunktionelles Enzym (die Polymerase- und die 3'-5' Exonuklease-Aktivität gehören zu der gleichen Polypeptidkette und können nur proteolytisch abgespalten werden), besitzt auch andere, bis jetzt unbekannte Fähigkeiten. Es desaminiert 2'-Desoxyadenosin zu 2'-Desoxyinosin (Beispiel 13) und reduziert ADP zu dADP in Anwesenheit von dGTP und NADPH. Als Nucleosid-Diphosphat-Kinase ist das multifunktionelle Enzym aus *E. coli* ebenfalls aktiv. Diese letzte Aktivität wurde auch von A. Kornberg (17) beobachtet.

Ähnliche Eigenschaften zeigt die DNA-Polymerase aus *Micrococcus luteus* (Sigma Chemie GmbH, Bestellnummer D 2626) (7), die Adenosin zu desaminieren und phosphorylieren vermag (Beispiel 14 und 15). Die Reduktion von CTP wurde spektrophotometrisch (Absorptionsabnahme von NADPH bei 340 nm) und durch HPLC-Analysen verfolgt.

Kaninchen-Antiserum gegen V 2 und V 3 zeigte eine positive Kreuzreaktion mit der *E. coli* DNA-Polymerase I in einem ELISA-Test auf Mikrotiterplatten.

Die immunologische Affinität zwischen V 3 und Polymerase I ist sehr groß. Strukturell sind die zwei Enzyme auch sehr ähnlich: sie bestehen aus einem Monomer und besitzen mehrere aktive Zentren bzw. Aktivitäten als Bestandteil eines einzigen Polypeptides. Immunoglobuline G gegen V 2 zeigen auch in gewissem Maße positive Kreuzreaktion mit der Polymerase I.

Dieses Phänomen (die Assoziation der vorgenannten Aktivitäten) bestätigt sich auch bei anderen Organismen und scheint universell zu sein.

So wurden zum Beispiel im Kalbsdarm schon vier Adenosin-Desaminasen nachgewiesen (20), die Bestandteil der beschriebenen multifunktionellen Enzyme sind und wahrscheinlich auch die anderen Aktivitäten besitzen wie die Adenosin-Desaminase aus Kalbsmilz.

Unveröffentlichte Ergebnisse von Untersuchungen des 2', 3'-Didesoxyadenosin-Metabolismus in menschlichen Zellen unterstützen meine Hypothese. Bei Desoxycytidin-Kinase- und Desoxyadenosin-Kinase-defizienten T-Lymphozyten wird eine Desaminierungsrate von ddA in Höhe von nur 25 % gegenüber dem Wildtyp registriert. Dies kann wie folgt erklärt werden: die dCK⁻ und dAK⁻ Zellen sind in zwei multifunktionellen Enzymen mit allen ihren Aktivitäten (Kinase, Reduktase, Desaminase, Polymerase) defizient, was die niedrige Desaminierungsrate erklärt.

Die Entdeckung der multifunktionellen Enzyme, die an der DNA-Replikation beteiligt sind, kann auch folgende Tatsache erklären: die virale T 7 DNA-Polymerase benötigt ein Thioredoxin-Molekül aus der Wirtszelle (*E. coli*), um überhaupt aktiv zu werden. Das Thioredoxin fungiert auch in diesem Falle als Redox-Coenzym für die Reduktion der Nucleotide. Warum eine DNA-Polymerase ein Thioredoxin-Molekül benötigt, war A. Kornberg nicht erklärlich (17, 18).

Eine Primase-Aktivität der DNA-Polymerase a aus menschlichen Zellen wurde schon nachgewiesen. Deswegen wird zur Zeit das Enzym "Polymerase-Primase" genannt. Der Name beschreibt nur zwei von allen Aktivitäten, die das Enzym besitzt (19).

Die 3'-5'-Exonuclease-Aktivität bei manchen solchen multifunktionellen Enzymen ist schon bekannt.

Es ist auch bekannt, daß es außergewöhnliche Schwierigkeiten während des Aufreinigungsvorgangs dieser Enzyme wegen unspezifischer Proteolyse und Aggregatebildung gibt. Die Aggregatebildung unterstützt in vivo die Allosterie dieser regulatorischen Enzyme (8, 9, 10, 12, 13, 17).

Die gewöhnlichen Proteaseinhibitoren, wie Phenylmethylsulfonylfluorid, beeinflussen nicht die unspezifische Proteolyse. Spezielle Inhibitoren, wie Antipain, Leupeptin, Aprotinin, etc. (12), inhibieren sowohl die Protease- als auch die Polymerase-Aktivität der multifunktionellen Enzyme.

Die reverse Transkriptase (RNA-abhängige-DNA-Polymerase) ähnelt sehr anderen Polymerasen und besitzt sogar DNA-abhängige-Polymerase-Aktivität. Es ist ein Fusionsprotein, zur Zeit Integrase genannt, das Protease-, reverse Transkriptase-, RNase H-und Endonuclease-Aktivität zeigt (21, 22). Sogar die sechs hochkonservierten funktionellen Regionen der DNA-Polymerasen sind auch bei den reversen Transkriptasen zu identifizieren (22).

Diese Fakten lassen vermuten, daß alle diese multifunktionellen Enzyme sich von einem ursprünglich gemeinsamen Gen entwickelt haben (16).

Anwendungen der Multifunktionalität der Enzyme V 1, V 2 und V 3

Die verschiedenen Aktivitäten der multifunktionellen Enzyme können einzeln für Produktionszwecke der basenmodifizierten Nucleoside, Nucleotide und Poly- oder Oligonucleotide angewendet werden. Die Substratspezifizität der *Lactobacillus*-Enzyme ist in fast allen Aktivitäten wesentlich breiter im Vergleich zu den entsprechenden Enzymen aus *E. coli* oder Kalbsmilz. Bei denen erfolgt die Reduktion der Nucleotide nur auf der Diphosphatstufe und sie besitzen nur Nucleosiddiphosphat-Kinase-Aktivität. Nur wenige Organismen besitzen multifunktionelle Enzyme mit einem solch breiten Substratspektrum.

Der sich aus der Multifunktionalität ergebende Vorteil ist die Möglichkeit der Durchführung einer ganzen Reaktionssequenz, wie sie in vivo mit hoher Effizienz mit Hilfe eines solchen Enzyms auch stattfindet.

Zum Beispiel kann die Nucleosid-Kinase-Aktivität entweder mit der de novo-Polymerase-Aktivität oder mit der Polymerisation eines Templates gekoppelt werden. Die de novo-Polymerisation erfolgt spontan nach der Phosphorylierung nur durch eine verlängerte Inkubation bei 35°C. Dabei entstehen Heteropolymere (Beispiel 16 und 17).

Die Herstellung von Homopolymeren erfolgt durch die de novo-Polymerisation eines einzigen Nucleotids (Beispiel 9). Die Enzyme V 2 und V 3 ermöglichen die Verknüpfung der Nucleotide auf allen Phosphorylierungsstufen.

Im Falle der Phosphorylierung eines Nucleosids gefolgt von der Polymerisation eines Templates werden die Substrate der zweiten Reaktion dem Enzym erst angeboten, wenn die erste Reaktion das Gleichgewicht schon erreicht hat (Beispiel 18).

Die Desoxyribose-Transferreaktion kann gut mit der Phosphorylierung und Polymerisationsreaktionen kombiniert werden.

Die Reduktion der Nucleotide kann auch nach der Phosphorylierung erfolgen (Beispiel 19).

Die wichtigsten Anwendungen der Enzyme V 1, V 2 und V 3, die sich durch hohe Wirtschaftlichkeit auszeichnen, sind :

- Die Synthese von neuen 2', 3'-Didesoxyribofuranoside, die bis jetzt noch nicht herstellbar waren.

- Die effiziente Phosphorylierung der 2', 3'-Didesoxyribonucleoside bis zu den Triphosphaten, die bis jetzt nur durch ein teures chemisches und enzymatisches Verfahren möglich war.

- Die enzymatische Synthese von Homo- und Heteropolynucleotiden. Von Vorteil bei der Herstellung der Homopolymeren durch de novo-Polymerisation ist, daß man keinen Primer für die Initiierung der Synthese braucht und das Substrat zu 100 % verbraucht wird, was die Aufreinigung des Produktes erheblich vereinfacht.

- Der Einsatz von V 3 (oder V 1 oder V 2) in der DNA-Sequenzier-Methode, der sogenannten Didesoxy-Methode nach Sanger (23).

- Die Tatsache, daß die *Lactobacillus*-Enzyme die Fähigkeit haben, ddNDP oder ddNMP (statt teures ddNTP) einzubauen, hat große wirtschaftliche Vorteile. Durch das Human-Genom-Sequenzierungsprogramm gewinnt diese Anwendung zusätzlich eine besondere Bedeutung.

- Durch wiederholte Verwendung eines einzigen biologischen Katalysators für eine Vielfalt von Reaktionen werden die Herstellungskosten der Endprodukte erheblich gesenkt.

Folgende Tatsachen ermöglichen die Synthese einer breiten Palette von modifizierten Nucleosiden, Nucleotiden sowie Polynucleotiden, welche bis jetzt noch nicht synthetisiert werden konnten:

a) das breite Substratspektrum der drei multifunktionellen Enzyme aus *Lactobacillus leichmannii,*

b) die Substratspezifizität der multifunktionellen Enzyme aus anderen prokaryontischen und eukaryontischen Zellen,

c) die unerwarteten Spezifitäten neuer enzymatischer Aktivitäten, die anhand dieser Theorie gezielt und systematisch nachgewiesen werden können.

Die Erfindung wird durch die Beispiele weiter erläutert:

Beispiel 1

150 Liter MRS-Medium wurden mit 1 Liter einer logarithmischen Vorkultur von *Lactobacillus leichmannii* (DSM 20076) angeimpft. Die Mikroorganismen wurden anaerob bei 37°C gerührt, bis sie die stationäre Phase erreicht haben (ca. 6 Stunden). Mit einer Cross-Flow-Filtrationsanlage wurde die Zellsuspension eingengt und danach abzentrifugiert. Die Ausbeute betrug 12,5 g/l Zellenfeuchtgewicht.

| MRS-Medium | |
|---|---|
| Pepton für Bakteriologie | 10 g |
| Fleischextrakt | 5 g |
| Hefeautolysat | 5 g |
| $K_2HPO_4$ | 2 g |
| Natriumcitrat | 2 g |
| Natriumacetat-trihydrat | 6 g |
| $MgSO_4•7H_2O$ | 0,58 g |
| $MnSO_4•4H_2O$ | 0,28 g |
| Tween 80 | 1 g |
| Glucose | 20 g |
| Wasser ad 1 Liter, eingestellt mit Salzsäure 1 N auf pH 6,2 bis 6,3. | |

20 g Zellen (Feuchtgewicht) wurden in 30 ml Aufschlußpuffer und 80 g Glasperlen (Durchmesser 0,75 mm) bei 4000 rpm in einem Glasperlendesintegrator aufgeschlossen. Der Aufschluß dauerte nicht länger als 5 Minuten.

| Aufschlußpuffer | |
|---|---|
| Phosphatpuffer (Na) | 20 mM, pH 6,5 |
| NaCl | 50 mM |
| EDTA | 0,1 mM |
| Mercaptoethanol | 1 mM |
| PMSF (Phenylmethylsulfonylfluorid) | 20 μM |

1. Schritt:

Der erhaltene Rohextrakt mit einer optimalen Proteinkonzentration von 10 mg/ml wurde unter Rühren bei 4°C mit 1 % (w/w) Protaminsulfatlösung, pH 6,5, eine Stunde vermischt, bis eine Endkonzentration an Protaminsulfat von 0,4 bis 0,45 % erreicht ist. Die ausgefallenen Proteine wurden bei 11.000 rpm 15 Minuten lang abzentrifugiert. Bei dieser Fällung werden größere Aggregate der Proteine gebildet, wodurch eine 30 %ige Aktivitätserhöhung von V 2 bewirkt wird.

2. Schritt:

Danach folgte eine erste Ammoniumsulfatfällung zwischen 0 bis 50 % Sättigung; hierauf schloß sich eine zweite Ammoniumsulfatfällung zwischen 50 bis 70 % Sättigung an. In dem letzten Sättigungsbereich fallen die Transferasen aus.

3. Schritt:

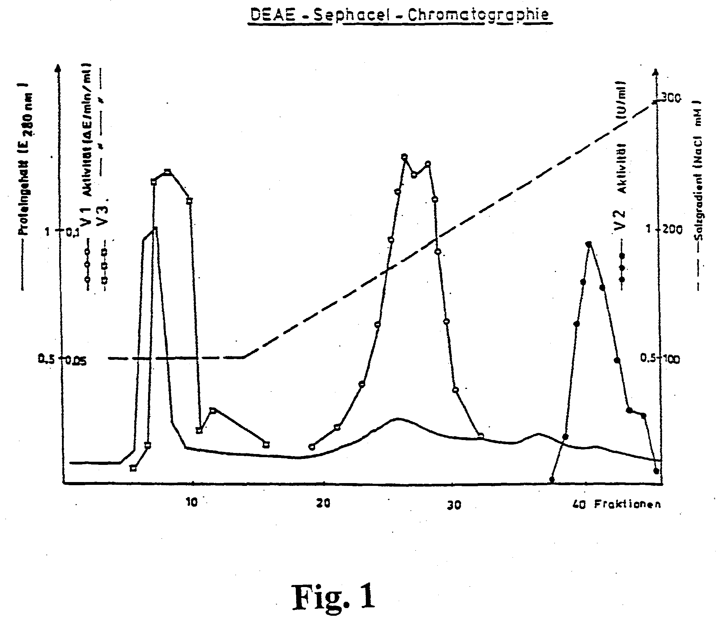

Die Nucleosid-Desoxyribosyltransferasen wurden auf einer DEAE-Sephacel-Säule, welche mit 20 mM Phosphatpuffer, 100 mM NaCl, 0,1 mM EDTA, 1 mM Mercaptoethanol äquilibriert war, voneinander getrennt. Im NaCl-Gradient wurden V 1 zwischen 160 bis 190 mM, V 2 zwischen 260 bis 280 mM und V 3 am Ende des Durchbruchs bei 100 mM NaCl eluiert; vgl. Fig. 1, Elutionsprofil.

4. Schritt:

Die erhaltenen einzelnen Fraktionen mit Transferaseaktivität wurden vereinigt und die erhaltenen drei Pools separat gegen 20 mM Tris-HCl, pH 7,0, 20 mM HCl, 4,0 mM DTT, 4,0 mM $MgCl_2$, 0,1 mM EDTA dialysiert. 3 ml 5'-AMP-Agarose (Affinitätsmatrix) wurden in eine Säule gepackt und mit dem genannten Puffer äquilibriert. Die V 3-haltige Probe (5 ml) wurde mittels einer peristaltischen Pumpe eine Stunde durch die Säule rezirkuliert (Fließgeschwindigkeit: 10 ml/Stunde). Die ungebundenen Proteine wurden mit 4 Säulenvolumina 100 mM KCl in dem gleichen Puffer weggewaschen. V 3 wird erst bei einer Konzentration von 300 mM KCl in dem Tris-HCl Puffer eluiert.
Durch dieses Aufreinigungsverfahren konnte die spezifische Aktivität von V 3 auf 20 E/mg Protein erhöht werden.

Damit liegt ein homogenes Protein vor.

Pro Gramm Zellenfeuchtgewicht konnten folgende Mengen Enzym aus den Zellen isoliert werden:

| V1 | 50 µg (1 Einheit) |
|---|---|
| V2 | 160 µg (2,7 Einheiten) |
| V3 | 550-600 µg (11-12 Einheiten) |

Beispiele für die Nucleosid-Transferase-Aktivität von V 3

Beispiel 2

Phosphatpuffer (Na) 20 mM, NaCl 50 mM, pH 6,2

| 2',3'-Didesoxyadenosin | 0,2 mM |
|---|---|
| 1,7-Dimethylguanin | 0,1 mM |
| V 3 | 10 E/ml Reaktionsansatz |

Temperatur: 35-37 °C
Dauer der Reaktion bis zum Gleichgewicht: 3-5 Tage
Endprodukt: 1,7-Dimethylguanin-9-$\beta$-D-2',3'-didesoxyribofuranosid

Hier wird wahrscheinlich die ddr sowohl in die Position N9 als auch in die Position N3 überführt.

In der gleichen Weise wird ein 2',3'-Didesoxyribosylrest auf:

1-Hydroxy-isoguanin
8-Methylxanthin
2-Hydroxypurin
1-Methyladenin
7-Methylguanin

übertragen.

Beispiel 3

Phosphatpuffer (Na) 20 mM, NaCl 50 mM, pH 6,2

| 2'-Desoxyinosin | 2 mM |
|---|---|
| 8-Bromguanin | 0,5 mM |
| V 3 | 5 E/ml Reaktionsansatz |

Temperatur: 35-37°C
Dauer der Reaktion: 24-30 Stunden
Endprodukt: 8-Bromguanosin

Beispiele für die Nucleosid- oder Nucleotid-Kinase-Aktivität von V 1, V 2 und V 3

Beispiel 4

Puffer: Tris-HCl 50 mM, pH 7,2

| $MgCl_2$ | 6,5 mM |
|---|---|
| Dithiothreitol (DTT) | 2,5 mM |
| Adenosin-5'-triphosphat | 5 mM |
| 2'-Desoxythymidin | 3 mM |
| V 3 | 5 E/ml Reaktionsansatz |

Temperatur: 35-37 °C
Endprodukte: dTMP, dTDP, dTTP (in Spuren).

Beispiel 5

| | |
|---|---|
| 2'-Desoxycytidin-5'-triphosphat | 5 mM |
| 2',3-Didesoxyadenosin | 3 mM |
| Puffer: Tris-HCl 50 mM, NaCl 50 mM, pH 7,2 | |
| $MgCl_2$ | 6,5 mM |
| DTT | 2,5 mM |
| V 3 | 10 E/ml Reaktionsansatz |

Temperatur: 35-37 °C
Das Gleichgewicht wird in 5-7 Tagen erreicht.
Endprodukte: ddAMP, ddADP, ddATP

In der gleichen Weise werden

1,7-Dimethylguanin-9-β-D-2',3'-didesoxyribofuranosid
1-Hydroxy-isoguanin-9-β-D-2',3'-didesoxyribofuranosid
8-Methylxanthin-9-β-D-2',3'-didesoxyribofuranosid
2-Hydroxypurin-9-β-D-2',3'-didesoxyribofuranosid
1-Methyladenin-9-β-D-2',3'-didesoxyribofuranosid
7-Methylguanin-9-β-D-2",3'-didesoxyribofuranosid

phosphoryliert.

Beispiel für die Ribonucleotid-Reduktase-Aktivität von V 1, V 2 und V 3

Beispiel 6

| | |
|---|---|
| Puffer: Natriumacetat | 60 mM |
| $K_2HPO_4$ | 3 mM |
| ATP (oder ADP oder AMP) | 1 mM |
| Coenzym B 12 | 4 $\mu$M |
| DTT | 30 mM |
| dGTP (Effektor) | 1 mM |
| Enzym (V 1,V 2 oder V 3) | 0,2-0,5 E/ml Reaktionsansatz |

Temperatur: 35°C
Dauer der Reaktion: 2-3 Tage
Endprodukt: dATP (dADP bzw. dAMP)

Beispiele für die Adenosin-Desaminase-Aktivität von V 1, V 2 und V 3

Beispiel 7

Phosphatpuffer 20 mM, NaCl 50 mM, pH 6,5

| | |
|---|---|
| 2'-Desoxycytidin | 2 mM |
| V 2 | 0,5 E/ml Reaktionsansatz |

Endprodukt: 2'-Desoxyuridin

Beispiel 8

Phosphatpuffer 20 mM, NaCl 50 mM, pH 6,5

| 2',3'-Didesoxyadenosin V 3 | 2 mM 5 E/ml Reaktionsansatz |
|---|---|

Endprodukt: 2',3'-Didesoxyinosin
Die Reaktionsansätze müssen bei 37°C über längere Zeit inkubiert werden, bevor die Desaminierung stattfindet. Die Dauer für die Desaminierungsreaktionen beträgt 2 bis 4 Tage.

Beispiele für die DNA-Polymerase-Aktivität von V 1, V 2 und V 3

Beispiel 9

de novo-Polymerisation

Puffer: Tris-HCl 60 mM pH 7,2

| DTT | 2,5 mM |
|---|---|
| dATP | 5 mM |
| Enzym: V 3 oder V 2 | 5 E/ml(1 E/ml) Reaktionsansatz |

Temperatur: 35°C
Dauer der Reaktion: 3 Tage
Produkt: Poly(dA)

Beispiel 10

Replikation der Poly [d(A-T)]

Puffer: Tris-HCl 40 mM, pH 7,55

| MgCl$_2$ | 5 mM |
|---|---|
| DTT | 1 mM |
| BSA (Rinderserumalbumin) | 50 µg/ml |
| Poly [d(A-T)] | 1,5 A$_{260}$ U |
| dATP | 1 mM |
| dTTP | 1 mM |
| dGTP | 1 mM |
| dCTP | 1 mM |
| Enzym: V 1, V 2 oder V 3 | 2-5 E/ml Reaktionsansatz |

Temperatur: 35-37°C
Dauer der Reaktion: 3-5 Tage
Produkt: Poly [d(A-T)]

Beispiel 11

Puffer: Tris-HCl 40 mM pH 7,2

| DTT | 2,5 mM |
|---|---|
| dAMP | 1 mM |
| MgCl$_2$ | 6,5 mM |
| BSA (Rinderserumalbumin) | 50 µg/ml |

(fortgesetzt)

| Poly [d(A-T)] | $1,5\ A_{260}\ U$ |
|---|---|
| dTTP | 1 mM |
| dGTP | 1 mM |
| dCTP | 1 mM |
| Enzym: V 3 | 5 E/ml Reaktionsansatz |

Temperatur: 35°C
Dauer der Reaktion: 3-5 Tage
Produkt: Poly [d(A-T)]

Beispiele für die Multifunktionalität ähnlicher Enzyme aus anderen Organismen

Beispiel 12

Puffer: Tris-HCl 100 mM, pH 7,5

| dAMP | 1 mM |
|---|---|
| dCTP | 2 mM |
| $MgCl_2$ | 10 mM |
| DTT | 1 mM |
| BSA (Rinderserumalbumin) | 100 µg/ml |
| Enzym: Adenosin-Desaminase aus Kalbsmilz | 2 E/ml Reaktionsansatz |

Temperatur: 35°C
Dauer der Reaktion: 24-30 Stunden
Produkt: dADP, dATP

Beispiel 13

Puffer: Phosphat 50 mM, pH 6,2

| dA | 2 mM |
|---|---|
| Enzym: DNA-Polymerase I aus E.coli | 2 E/ml Reaktionsansatz |

Temperatur: 37°C
Dauer der Reaktion: 5 Tage
Produkte: dI, Hypoxanthin

Beispiel 14

Puffer: Phosphat 50 mM, pH 6,2

| dA | 2 mM |
|---|---|
| BSA (Rinderserumalbumin) | 50 µg/ml |
| Enzym: DNA-Polymerase aus Micrococcus luteus | 1 E/ml Reaktionsansatz |

Temperatur: 35°C
Dauer der Reaktion: 20-24 Stunden
Produkte: dI, Hypoxanthin

Beispiel 15

Puffer: Tris-HCl 40 mM, pH 7,5

| dA | 3 mM |
|---|---|
| dCTP | 5 mM |
| MgCl$_2$ | 5 mM |
| DTT | 2,5 mM |
| BSA (Rinderserumalbumin) | 50 μg/ml |
| Enzym: DNA-Polymerase aus Micrococcus luteus | 1 E/ml Reaktionsansatz |

Temperatur: 35°C
Dauer der Reaktion: 2 Tage
Produkte: dAMP, dADP, dATP

Beispiele für Reaktionssequenzen

Beispiel 16

Puffer: Tris-HCl 40 mM, pH 7,55

| MgCl$_2$ | 6,5 mM |
|---|---|
| DTT | 2,5 mM |
| dATP | 5 mM |
| dC | 3 mM |
| Enzym: V 3 | 5 E/ml Reaktionsansatz |

Temperatur: 35°C
Dauer der Reaktion: 2 Tage
Produkt: dCMP, dCDP
Eine spontane de novo-Polymerisation erfolgt in den nächsten 3 Tagen.
Produkt: Poly (dA-dC)

Beispiel 17

Puffer: Tris-HCl 40 mM, pH 7,55

| MgCl$_2$ | 6,5 mM |
|---|---|
| DTT | 2,5 mM |
| dATP | 5 mM |
| dG | 3 mM |
| Enzym: V 2 | 1 E/ml Reaktionsansatz |

Temperatur: 35°C
Dauer der Reaktion: 2 Tage
Nach der de novo-Polymerisation entsteht Poly (dA-dG).

Beispiel 18

Puffer: Tris-HCl 40 mM, pH 7,2

| dA | 3 mM |
|---|---|
| dCTP | 5 mM |
| MgCl$_2$ | 6,5 mM |
| DTT | 2,5 mM |
| BSA (Rinderserumalbumin) | 50 μg/ml |
| Enzym: V 3 | 5 E/ml Reaktionsansatz |

(fortgesetzt)

| Nach 3 Tagen wurde dazu gegeben: | |
|---|---|
| Poly [d(A-T)] | 1,5 $A_{260}$ U |
| dGTP | 1 mM |
| dTTP | 1 mM |

Temperatur: 35°C
Dauer der Reaktion: 5-6 Tage
Produkte: dAMP, dADP, dATP, Poly [d(A-T)]

Beispiel 19

Puffer: Tris-HCl 50 mM, pH 7,5

| AMP | 1 mM |
|---|---|
| dGTP | 2 mM |
| Coenzym B 12 | 4 $\mu$M |
| DTT | 30 mM |
| BSA (Rinderserumalbumin) | 50 $\mu$g/ml |
| Enzym: V 3 | 5 E/ml Reaktionsansatz |

Nachdem die AMP-Reduktion zu dAMP das Gleichgewicht erreicht hat, wird

| MgCl$_2$ | 5 mM zugegeben |
|---|---|

Produkte: dAMP, dADP, dATP

Literaturverzeichnis

(1)    Durham J.P., Ives D.H.
       Biochim. Biophys. Acta 228, 9-25, (1971)
(2)    Deibel M.R., Reznik R.B., Ives D.H.
       J. Biol. Chem. 252 (22), 8240-44, (1977)
(3)    Deibel M.R., Ives D.H.
       J. Biol. Chem. 252 (22), 8235-38, (1977)
(4)    Ikeda S., Chakravarty R., Ives D.H.
       J. Biol. Chem. 261 (34), 15836-43, (1986)
(5)    Chakravarty R., Ikeda S., Ives D.H.
       Biochemistry 23, 6235-40, (1984)
(6)    Kirshner K., Bisswanger H.
       Annu. Rev. Biochem. 45, 143-66, (1976)
(7)    Harper & Row
       Proc. Acid. Res., 284, (1966)
(8)    Lee M.Y.W.T.
       Biochemistry 27, 5188-93, (1988)
(9)    Hübscher U.
       Experientia 39 (1), 1-26, (1983)
(10)   Kaguni L.S., Lehman I.R.
       Biochim. Biophys. Acta 950, 87-101, (1988)
(11)   Hübscher U.
       TIBS September 1984, 390-93
(12)   Heilbronn R., Schlenhofer J.R.
       Int. J. Cancer 36, 85-91, (1985)
(13)   Focher F., Spadari S., Ginelli B.
       Nucl. Acid Res. 16 (14), 6279-95, (1988)

(14)  Pfrogner N.
      Arch. Biochim. Biophys. 119, 141-46, (1967)
(15)  Bernard A., Zaballos A., Salas M., Blanco L.
      EMBO J. 13(6), 4219-25, (1987)
(16)  Wong S.W., Wahl A.F., Yuan N.A., Arai N., Pearson B.E.,
      Arai K., Korn D., Hunkapiller M., Wang T.S.F.
      EMBO J. 7 (1), 37-47, (1988)
(17)  Kornberg A.
      DNA Replication ( 1980), 105 und 125-127
      Freeman W.H. and Co., San Francisco
(18)  Kornberg A.
      Supplement in DNA Replication (1982)
      Freeman W.H. and Co., San Francisco
(19)  Bialek G., Nasheuer H.P., Goetz H., Behnke B., Grosse F.
      Biochim. Biophys. Acta 951, 290-97, (1988)
(20)  Brady T.G., O'Connel W.O.
      Biochim. Biophys. Acta 62, 216 (1962)
(21)  Oxford J.S., Coates A.R.M., Sia D.I., Brown K., Asad S.
      Journal of Antimicrobial Chemotherapy 23, Suppl.A, 9-27, (1989)
(22)  Tisdale M., Larder B.A., Loewe D.M.,Stammers D.J., Purifoy
      D.J.M., Ertl P., Bradley C., Kemp S., Darby G.K., Powell K.L.
      Journal of Antimicrobial Chemotherapy 23, Suppl.A, 47-54 (1989)
(23)  Sanger F., Nicklen S., Golson A.R.
      Proc. Nat. Acad. Sci. USA 74, 5463-67, (1977)

**Patentansprüche**

1.  Verfahren zur Herstellung von Desoxyribonucleotiden, dadurch gekennzeichnet, daß man ein Ribonucleotid als Substrat mit einem Enzym, das mindestens folgende Aktivitäten aufweist, nämlich Nucleosid-Desoxyribosyltransferase-, Nucleosid- oder Nucleotid-Kinase-, Ribonucleotid-Reduktase-, Adenosin-Desaminase- und DNA-Polymerase-Aktivität, in wässrigem Medium in Kontakt bringt und das gebildete Desoxyribonucleotid aus dem Reaktionsgemisch abtrennt.

2.  Verfahren nach Anspruch 1, dadurch gekennzeichnet, daß man eine Nucleosid-Desoxyribosyltransferase aus *Lactobacillus leichmannii* verwendet.

3.  Verfahren nach Anspruch 2, dadurch gekennzeichnet, daß man die Reaktion in Gegenwart von Coenzym B12 durchführt.

4.  Verfahren nach Anspruch 2, dadurch gekennzeichnet, daß man die Reaktion in Gegenwart eines Desoxyribonucleosidtriphosphats als Effektor durchführt.

5.  Verfahren nach Anspruch 2, dadurch gekennzeichnet, daß man die Reaktion in Gegenwart von Coenzym B12 und eines Desoxyribonucleosidtriphosphats als Effektor durchführt.

6.  Verfahren zur Herstellung von Desoxyribonucleotiden, dadurch gekennzeichnet, daß man ADP oder UDP als Substrat mit Adenosin-Desaminase aus Kalbsmilz in wässrigem Medium, welches Effektoren und NADPH enthält, in Kontakt bringt und das gebildete Desoxyribonucleotid aus dem Reaktionsgemisch abtrennt.

7.  Verfahren zur Herstellung von Desoxyribonucleotiden, dadurch gekennzeichnet, daß man ein CTP als Substrat mit DNA-Polymerase aus *Micrococcus luteus* in wässrigem Medium in Kontakt bringt und das gebildete Desoxyribonucleotid aus dem Reaktionsgemisch abtrennt.

8.  Verfahren zur Herstellung von Desoxyribonucleotiden, dadurch gekennzeichnet, daß man ADP als Substrat mit DNA-Polymerase I aus *E. coli* in wässrigem Medium, welches GTP und NADPH enthält, in Kontakt bringt und das gebildete Desoxyribonucleotid aus dem Reaktionsgemisch abtrennt.

**9.** Verfahren zur Herstellung von Phosphatestern von Nucleosiden, dadurch gekennzeichnet, daß man ein Nucleosid oder Nucleotid als Substrat mit einem Enzym, das mindestens folgende Aktivitäten aufweist, nämlich Nucleosid-Desoxyribosyltransferase-, Nucleosid- oder Nucleotid-Kinase-, Ribonucleotid-Reduktase-, Adenosin-Desaminase- und DNA-Polymerase-Aktivität, in wässrigem Medium und in Gegenwart eines Nucleotids in Kontakt bringt und den gebildeten Phosphatester aus dem Reaktionsgemisch abtrennt.

**10.** Verfahren nach Anspruch 9, dadurch gekennzeichnet, daß man eine Nucleosid-Desoxyribosyltransferase aus *Lactobacillus leichmannii* verwendet.

**11.** Verfahren nach Anspruch 10, dadurch gekennzeichnet, daß man als Nucleosid ein Desoxyribonucleosid verwendet.

**12.** Verfahren nach Anspruch 10, dadurch gekennzeichnet, daß man als Nucleosid ein Didesoxynucleosid verwendet.

**13.** Verfahren zur Herstellung von Phosphatestern von Nucleosiden, dadurch gekennzeichnet, daß man dAMP oder dCMP als Substrat mit Adenosin-Desaminase aus Kalbsmilz in wässrigem Medium und in Gegenwart eines Nucleotids in Kontakt bringt und den gebildeten Phosphatester aus dem Reaktionsgemisch abtrennt.

**14.** Verfahren zur Herstellung von Phosphatestern von Nucleosiden, dadurch gekennzeichnet, daß man Adenosin oder dAdenosin als Substrat mit DNA-Polymerase aus *Micrococcus luteus* in wässrigem Medium und in Gegenwart eines Nucleotids in Kontakt bringt und den gebildeten Phosphatester aus dem Reaktionsgemisch abtrennt.

**Claims**

**1.** A process for the preparation of deoxyribonucleotides, characterized in that a ribonucleotide as substrate is contacted in an aqueous medium with an enzyme having at least the following activities, namely nucleoside deoxyribosyltransferase activity, nucleoside or nucleotide kinase activity, ribonucleotide reductase activity, adenosine deaminase activity and DNA polymerase activity, and the deoxyribonucleotide formed is separated from the reaction mixture.

**2.** The process according to claim 1, characterized in that a nucleoside deoxyribosyltransferase from *Lactobacillus leichmannii* is used.

**3.** The process according to claim 2, characterized in that the reaction is carried out in the presence of coenzyme $B_{12}$.

**4.** The process according to claim 2, characterized in that the reaction is carried out in the presence of a deoxyribonucleoside triphosphate as an effector.

**5.** The process according to claim 2, characterized in that the reaction is carried out in the presence of coenzyme $B12$ and a deoxyribonucleoside triphosphate as an effector.

**6.** A process for the preparation of deoxyribonucleotides, characterized in that ADP or UDP as substrate is contacted in an aqueous medium containing effectors and NADPH with adenosine deaminase from calf spleen and the deoxyribonucleotide formed is separated from the reaction mixture.

**7.** A process for the preparation of deoxyribonucleotides, characterized in that CTP as substrate is contacted in an aqueous medium with DNA polymerase from *Micrococcus luteus* and the deoxyribonucleotide formed is separated from the reaction mixture.

**8.** A process for the preparation of deoxyribonucleotides, characterized in that ADP as substrate is contacted in an aqueous medium containing GTP and NADPH with DNA polymerase I from *Escherichia coli* and the deoxyribonucleotide formed is separated from the reaction mixture.

**9.** A process for the preparation of phosphate esters of nucleosides, characterized in that a nucleoside or a nucleotide as substrate is contacted in an aqueous medium and in the presence of a nucleotide with an enzyme having at least the following activities, namely nucleoside deoxyribosyltransferase activity, nucleoside or nucleotide kinase activity, ribonucleotide reductase activity, adenosine deaminase activity and DNA polymerase activity, and the phosphate ester formed is separated from the reaction mixture.

10. The process according to claim 9, characterized in that a nucleoside deoxyribosyltransferase from *Lactobacillus leichmannii* is used.

11. The process according to claim 10, characterized in that as nucleoside a deoxyribonucleoside is used.

12. The process according to claim 10, characterized in that a dideoxynucleoside is used as a nucleoside.

13. A process for the preparation of phosphate esters of nucleosides, characterized in that dAMP or dCMP as substrate is contacted in an aqueous medium and in the presence of a nucleotide with adenosine deaminase from calf spleen and the phosphate ester formed is separated from the reaction mixture.

14. A process for the preparation of phosphate esters of nucleosides, characterized in that adenosine or deoxyadenosine as substrate is contacted in an aqueous medium and in the presence of a nucleotide with DNA polymerase from *Micrococcus luteus* and the phosphate ester formed is separated from the reaction mixture.

**Revendications**

1. Procédé pour la préparation de désoxyribonucléotides, caractérisé en ce qu'un ribonucléotide servant de substrat est mis en contact dans un milieu aqueux avec une enzyme ayant au moins les activités suivantes, à savoir une activité de nucléoside désoxyribosyltransférase, une activité de nucléoside ou nucléotide kinase, une activité de ribonucléotide réductase, une activité d'adénosine désaminase et une activité d'ADN polymérase, et le désoxyribonucléotide formé est séparé du mélange réactionnel.

2. Procédé selon la revendication 1, caractérisé en ce qu'une nucléoside désoxyribosyltransférase de *Lactobacillus leichmannii* est utilisée.

3. Procédé selon la revendication 2, caractérisé en ce que la réaction est effectuée en présence de coenzyme $B_{12}$.

4. Procédé selon la revendication 2, caractérisé en ce que la réaction est effectuée en présence d'un désoxyribonucléoside triphosphate en tant qu'effecteur.

5. Procédé selon la revendication 2, caractérisé en ce que la réaction est effectuée en présence de coenzyme $B_{12}$ et d'un désoxyribonucléoside triphosphate en tant qu'effecteur.

6. Procédé pour la préparation de désoxyribonucléotides, caractérisé en ce que de l'ADP ou de l'UDP servant de substrat est mis en contact dans un milieu aqueux contenant des effecteurs et du NADPH avec de l'adénosine désaminase de rate de veau et le désoxyribonucléotide formé est séparé du mélange réactionnel.

7. Procédé pour la préparation de désoxyribonucléotides, caractérisé en ce que du CTP servant de substrat est mis en contact dans un milieu aqueux avec de l'ADN polymérase de *Micrococcus luteus* et le désoxyribonucléotide formé est séparé du mélange réactionnel.

8. Procédé pour la préparation de désoxyribonucléotides, caractérisé en ce que de l'ADP servant de substrat est mis en contact dans un milieu aqueux contenant du GTP et du NADPH avec de l'ADN polymérase I *d'Escherichia coli* et le désoxyribonucléotide formé est séparé du mélange réactionnel.

9. Procédé pour la préparation de phosphate esters de nucléosides, caractérisé en ce qu'un nucléoside ou un nucléotide servant de substrat est mis en contact dans un milieu aqueux et en présence d'un nucléotide avec une enzyme ayant au moins les activités suivantes, à savoir une activité de nucléoside désoxyribosyltransférase, une activité de nucléoside ou nucléotide kinase, une activité de ribonucléotide réductase, une activité d'adénosine désaminase et une activité d'ADN polymérase, et le phosphate ester formé est séparé du mélange réactionnel.

10. Procédé selon la revendication 9, caractérisé en ce qu'une nucléoside désoxyribosyltransférase de *Lactobacillus leichmannii* est utilisée.

**11.** Procédé selon la revendication 10, caractérisé en ce qu'un désoxyribonucléoside est utilisé en tant que nucléoside.

**12.** Procédé selon la revendication 10, caractérisé en ce qu'un didésoxynucléoside est utilisé en tant que nucléoside.

**13.** Procédé pour la préparation de phosphate esters de nucléosides, caractérisé en ce que du dAMP ou du dCMP servant de substrat est mis en contact dans un milieu aqueux et en présence d'un nucléotide avec de l'adénosine désaminase de rate de veau et le phosphate ester formé est séparé du mélange réactionnel.

**14.** Procédé pour la préparation de phosphate esters de nucléosides, caractérisé en ce que de l'adénosine ou de la désoxyadénosine servant de substrat est mise en contact dans un milieu aqueux et en présence d'un nucléotide avec de l'ADN polymérase de *Micrococcus luteus* et le phosphate ester formé est séparé du mélange réactionnel.

EP 0 491 739 B1

DEAE - Sephacel - Chromatographie

Fig. 1